# EUROPEAN PATENT APPLICATION

(11) **EP 1 700 613 A1**
(43) Date of publication of application: **13.09.2006**
(21) Application number: 05100449.7
(22) Date of filing: 25.01.2005
(51) Int. Cl.: A61M 5/315, A61M 5/24

(54) **Injection device for administering a medication liquid**

(71) Applicant: Pfizer Health AB, 112 87 Stockholm (SE)
(72) Inventor: The designation of the inventor has not yet been filed
(74) Representative: Courgeon, Antoine

(57) **Abstract**

Injection device for administering multiple doses of a medication liquid, said device (1) comprising a housing (2), a cartridge (6) containing a preparation or precursor component for the preparation and having a front part and a rear part, the rear part being closed by a movable wall (10), a displacement of which wall causes the preparation to be moved forward the other end of the cartridge (6), through which the liquid exits, and actuating means (16) for actuating the wall (10) via a plunger (14) whose forward movement inside the housing (2) is driven by an actuator (16) at the proximal end of the housing (2) and adapted to be actuated by the user, said injection device (1) further comprises means (30) moving concomitantly with the plunger (14) and co-operating with a graduated scale (24) on the device (1) that is oblique to the axis of general forward movement of said plunger (14).

## Description

The present invention relates to an injection device for administering multiple doses of a medication liquid and provides a fast and simple way to check that the volume of the dose that one is preparing to administer to oneself or to another person corresponds to the programmed volume of the dose.

### Background of the invention

Injection devices of the above kind are known in the art. One such device conventionally comprises a generally elongate housing adapted to be held by a user and to a distal end of which is fixed an cartridge containing the liquid to be administered. The cartridge has an internal wall adapted to be moved by a plunger mobile axially inside the housing to expel a dose of liquid. Forward movement of the plunger inside the housing is driven by an actuator at a proximal end of the housing that can be operated by the user. The injection device further comprises a knurled knob carrying a graduated scale by means of which the user may adjust the volume of the dose to be injected in accordance with the medical prescription. To this end, the user must turn the knob to bring the appropriate graduation on the scale against a mark on the housing of the injection device. By doing this, the user programmes the movement of the plunger and therefore the volume of the dose to be injected.

When he wishes to administer an injection, the user must first arm the injection device by pulling out the actuator. During this rearward movement, the actuator moves partly out of the housing of the device and uncovers a second graduated scale formed of regularly spaced axial lines of varying length. As a function of the distance to which the actuator is pulled out, the lines are progressively concealed by the housing of the device, the last line visible indicating the volume of the dose that is ready to be injected. Thus by comparing the indications provided by the two graduations, the user is able to check that the volume of the dose he is preparing to administer corresponds to the programmed volume.

The major drawback of the above device is that the user finds it difficult to read the indications of the second graduated scale. This is because it is not easy to distinguish accurately between the last line that is still visible and the first line that is concealed by the housing of said device. Furthermore, it may be necessary to rotate the injection device on itself to uncover the line indicating the volume of the dose ready to be injected, which is inconvenient.

### Summary of the Invention

An object of the present invention is to remedy the problems mentioned above and other problems by providing an injection device providing a clear and accurate way of checking that the volume of the dose that is ready to be injected corresponds to the volume of the prescribed dose.

To this end, the present invention provides an injection device for administering multiple doses of a medication liquid, said device comprising a housing, a cartridge containing a preparation or precursor component for the preparation and having a front part and a rear part, the rear part being closed by a movable wall, a displacement of which wall causes the preparation to be moved forward the other end of the cartridge, through which the liquid exits, and actuating means for actuating the wall via a plunger whose forward movement inside the housing is driven by an actuator at the proximal end of the housing and adapted to be actuated by the user, said injection device being characterised in that it further comprises means moving concomitantly with the plunger and co-operating with a graduated scale on the device that is oblique to the axis of general forward movement of said plunger.

Thanks to these features, the injection device provides a clear, legible and accurate indication of the volume of the dose that is ready to be injected. This result is achieved because the graduated scale indicating the volume of the dose is oblique rather than parallel to the axis of general forward movement of the plunger. On each injection of a dose of medication liquid, the plunger moves forward too small a distance to be represented directly on a graduated scale parallel to its direction of forward movement. For this reason, the graduated scale is oblique to the direction of forward movement of the plunger, so that the movement of the means representing the forward movement of the plunger is projected onto said scale in accordance with its angle of inclination and is therefore demultiplied and thus easier to read.

In one embodiment of the invention, the injection device comprises a dosing ring carrying a dosing scale that is visible from the outside through an observation window in the housing of said injection device.

The user can therefore check before each injection that the indication provided by the dosing ring, which corresponds to his initial setting of the volume of the dose to be injected, coincides with the indication provided by the oblique graduated scale, which corresponds to the volume of the dose that is ready to be injected. This makes the device much safer to use, especially when preparing to inject the final dose of medication liquid, the volume of which usually does not correspond to the prescribed volume. Because the user is able to determine accurately the volume of liquid remaining in the cartridge, he can tell what dose to prepare with the injection device.

### Brief description of the drawings

Other features and advantages of the present invention will emerge more clearly from the following detailed description of one embodiment of an injection device of the invention, given by way of purely illustrative and non-limiting example and with reference to the appended drawings, in which:
- figure 1 is a general plan view of the injection device of the invention, partly in section at the level of the cartridge containing the liquid to be administered;

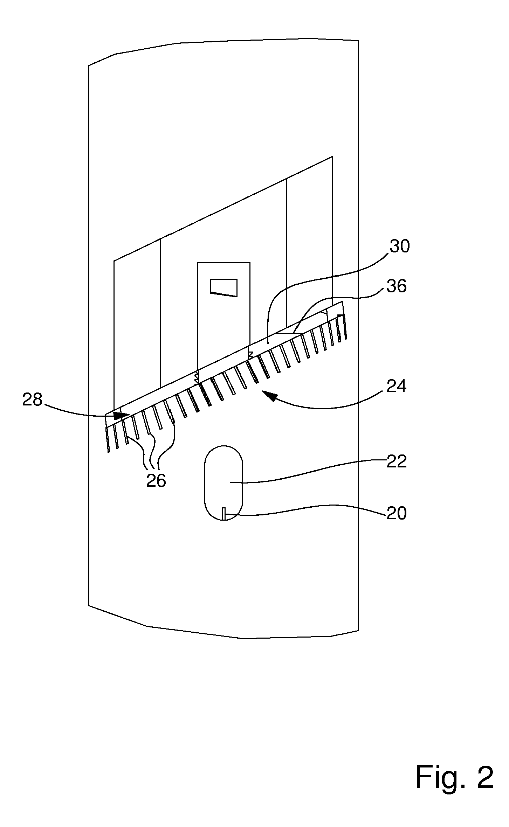

- figure 2 is a view to a larger scale of the ringed area in figure 1;
- figure 3 is a partially cutaway perspective view of the device of the invention; and
- figure 4 is a view to a larger scale of the ringed area in figure 3.

### Detailed description of the preferred embodiments

The present invention stems from the general inventive concept of projecting onto an oblique graduated scale the small linear displacements of the plunger of an injection device that causes the expulsion of a dose of medication liquid from an cartridge. Projected onto an oblique axis in this way, the movements of the plunger are demultiplied and are therefore very easy to read, which makes this kind of device much safer and much more convenient to use.

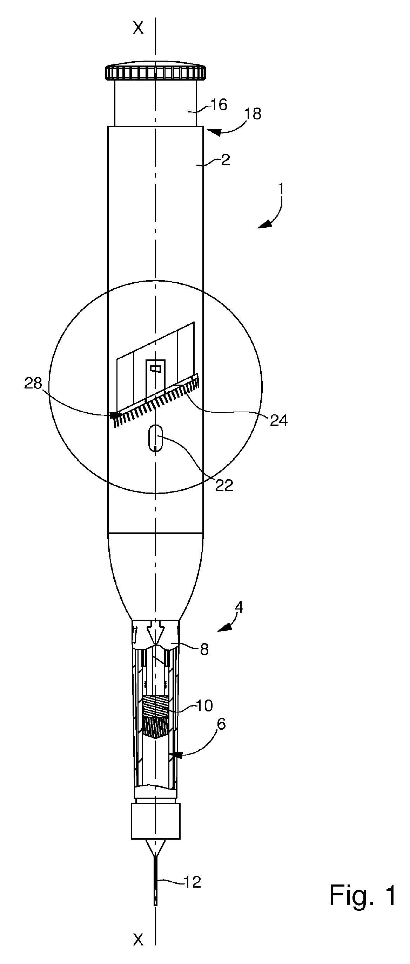

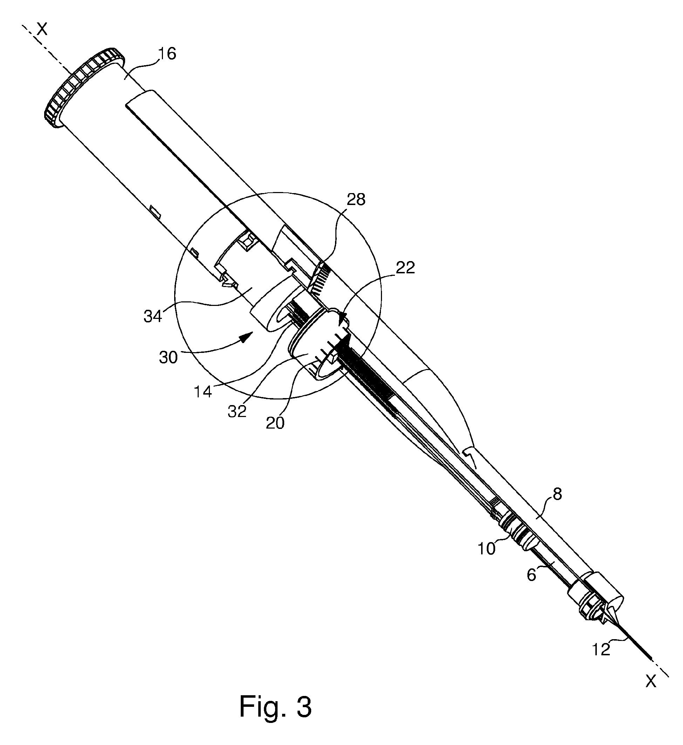

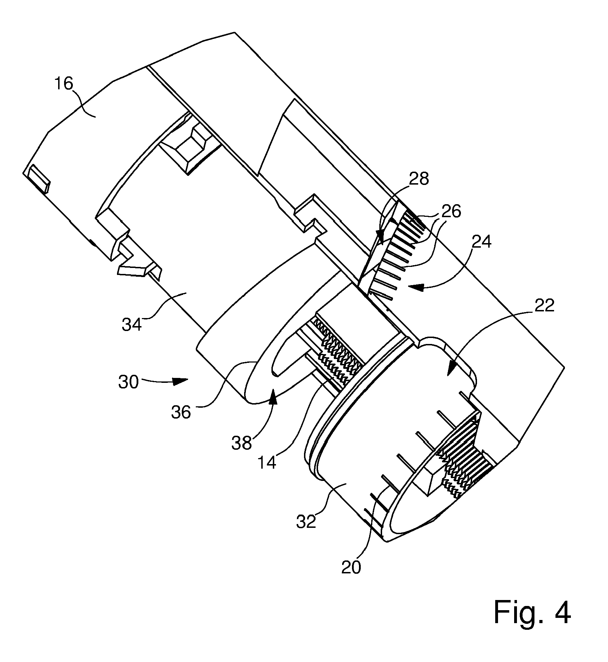

Figure 1 is a general plan view of the injection device 1 of the invention preferably adapted to be held by a user. The device 1 comprises a generally elongate housing 2. To a distal end 4 of the housing 2 is fixed a cartridge 6 protected by a plastic cap 8 and containing the medication liquid to be administered. The cartridge 6 has one end closed by a sealed wall 10 and adapted to be moved toward the other end of the cartridge 6, which is terminated by a needle 12 through which the liquid exits. The wall 10 is moved by a plunger 14 (see Figures 3 and 4) whose forward movement inside the housing 2 is driven by an actuator 16 at a proximal end 18 of the housing 2 and adapted to be operated by the user.

A first dosing scale 20 is visible from the outside through a first observation window 22 in the housing 2. This first dosing scale 20 enables the user to adjust the volume of the dose to be injected as a function of the medical prescription.

On the housing 2 is a second graduated scale 24 oblique to the general axis of symmetry X-X of the injection device 1. The scale 24 is rectilinear and is formed of a succession of parallel and regularly spaced segments or graduations 26. The scale 24 extends along a second observation window 28 in the housing 2 through which can be seen at least part of means 30 representative of the forward movement of the plunger 14, to be described in more detail below. The scale 24 enables the user to check that the volume of the dose he is preparing to inject corresponds to the volume of the dose that he programmed before using the device 1, and this makes the device extremely safe to use.

Figure 3 is a perspective view of the device 1 with the housing 2 partly cutaway and figure 4 is a view to a larger scale of the ringed area in figure 3. These two figures show that the first dosing scale 20 is on a dosing ring 32 that is prevented from moving in translation but mobile in rotation, to enable the user to move into the observation window 22 the indication corresponding to the volume of the dose prescribed for him.

The wall 10 (which can be seen in figures 1 and 3) is moved by the plunger 14, whose general direction of forward movement coincides with the axis of longitudinal symmetry X-X of the device 1. The forward movement of the plunger is driven by the actuator 16 via the means 30 representative of the forward movement of said plunger 14. The means 30 take the form of a forward movement bush 34 fastened to the actuator 16.

The device 1 operates in the following manner. To arm said device 1 and prepare it to dispense a new dose of liquid, the user pulls out the actuator 16 until it stops moving. The bush 34 is withdrawn concomitantly and remains partly visible from the outside through the second observation window 28, the intersection of the circular edge 36 of its front face 38 with one of the graduations 26 of the oblique scale 24 enabling the user to determine the volume of the dose he is preparing to administer. In a different embodiment, this reading could be effected by determining the intersection of a marker on the bush with the oblique scale 24 or on a part connected directly or indirectly to the plunger 14.

After arming the device 1, the user administers the injection by pushing on the actuator 16, which moves forward the bush 34 and therefore the plunger 14, which applies pressure to the wall 10 to expel a dose of medication liquid. The plunger 14 and therefore the bush 34 move forward in small increments, and it would be difficult or even impossible to represent those increments on a scale parallel to the direction of movement of those elements. The benefit of the invention is that, by projecting these distances onto a scale oblique to the general direction of forward movement of the plunger 14, the linear movements of the component 14 are amplified and made easy to read. The user can therefore check at any time that the volume of the dose ready to be injected corresponds to the volume that he programmed before using the device 1, which increases his confidence that the product is safe to use.

It goes without saying that the present invention is not limited to the embodiments that have just been described and that the person skilled in the art may envisage various simple modifications and variations that lie within the scope of the invention as defined by the appended claims.

## Claims

1. An injection device for administering multiple doses of a medication liquid, said device (1) comprising a housing (2), a cartridge (6) containing a preparation or precursor component for the preparation and having a front part and a rear part, the rear part being closed by a movable wall (10), a displacement of which wall causes the preparation to be moved forward the other end of the cartridge (6), through which the liquid exits, and actuating means (16) for actuating the wall (10) via a plunger (14) whose forward movement inside the housing is driven by an actuator (16) at the proximal end (18) of the housing (2) and adapted to be actuated by the user, said injection device (1) being **characterised in that** it further comprises means (30) moving concomitantly with the plunger (14) and co-operating with a graduated scale (24) on the device (1) that is oblique to the axis of general forward movement of said plunger (14).

2. An injection device according to claim 1, **characterised in that** the means (30) moving concomitantly with the plunger (14) comprise a forward movement bush (34) adapted to move forward and backward inside the housing (2) and through which forward movement of the actuator (16) is transmitted to the plunger (14).

3. An injection device according to claim 2, **characterised in that** the graduated scale (24) extends along an observation window (28) in the housing (2) through which the forward movement bush (34) is visible.

4. An injection device according to any of claims 1 to 3, **characterised in that** it comprises a dosing ring (32) carrying a dosing scale (20) that is visible from the outside through an observation window (22) in the housing (2) of said injection device (1) .

5. An injection device according to claim 4, **characterised in that** the dosing ring (32) is prevented from moving in translation inside the housing (2) but free to rotate before selection of the dose to be injected.
